# EUROPÄISCHE PATENTSCHRIFT

(11) **EP 3 793 973 B1**
(45) Veröffentlichungstag und Bekanntmachung des Hinweises auf die Patenterteilung: **28.12.2022**
(21) Anmeldenummer: 18727231.5
(22) Anmeldetag: 18.05.2018
(51) Int. Cl.: C07C 67/08, C07C 67/465, C07C 69/68, C07D 319/12, C08G 63/08, C08J 11/10

(54) **VERFAHREN ZUR HERSTELLUNG EINES INTRAMOLEKULAREN DIESTERS EINER HYDROXYCARBONSÄURE SOWIE VERFAHREN ZUR HERSTELLUNG EINER POLYHYDROXYCARBONSÄURE**
PROCESS FOR PRODUCING AN INTRAMOLECULAR DIESTER OF A HYDROXYCARBOXYLIC ACID AND PROCESS FOR PRODUCING A POLYHYDROXYCARBOXYLIC ACID.
PROCÉDÉ POUR PRODUIRE UN DIESTER INTRAMOLÉCULAIRE D'UN ACIDE HYDROCARBOXYLIQUE ET PROCÉDÉ POUR PRODUIRE UN ACIDE POLYHYDROXYCARBOXYLIQUE

(43) Veröffentlichungstag der Anmeldung: 24.03.2021
(73) Patentinhaber: Fraunhofer-Gesellschaft zur Förderung der angewandten Forschung e.V., 80686 München (DE)
(72) Erfinder: JESSE, Kathrin, 14478 Potsdam (DE); LIESKE, Antje, 14480 Potsdam (DE); HIRSEKORN, Max, 10557 Berlin (DE)
(74) Vertreter: Pfenning, Meinig & Partner mbB
(86) Internationale Anmeldenummer: PCT/EP2018/063130
(87) Internationale Veröffentlichungsnummer: WO 2019/219210

(56) Entgegenhaltungen:
- EP-A1- 1 310 496
- EP-A1- 1 484 311
- EP-A1- 2 559 725
- WO-A1-2015/086614

## Beschreibung

Die vorliegende Erfindung betrifft ein Verfahren zur Herstellung eines intramolekularen Diesters einer Hydroxycarbonsäure, bei dem eine Hydroxycarbonsäure in einem ersten Schritt zu einer oligomeren Hydroxycarbonsäure polykondensiert wird. Anschließend erfolgt eine cyclisierende Depolymerisation der in der ersten Stufe erhaltenen oligomeren Hydroxycarbonsäure, wobei der intramolekulare Diester entsteht. Die vorliegende Erfindung zeichnet sich dadurch aus, dass in die Stufe der cyclisierenden Depolimerisation ein Kunststoff-Recyclat aufgegeben wird. Das Kunststoffrecyclat beinhaltet dabei beispielsweise eine Polyhydroxycarbonsäure, die sich von der Hydroxycarbonsäure, die in der ersten Stufe eingesetzt wurde, ableitet. Ebenso kann das Kunststoffrecyclat eine Co-Polyhydroxycarbonsäure, oder einen Blend beinhaltend die zuvor genannten (Co-)Polyhydroxycarbonsäure (n) beinhalten.

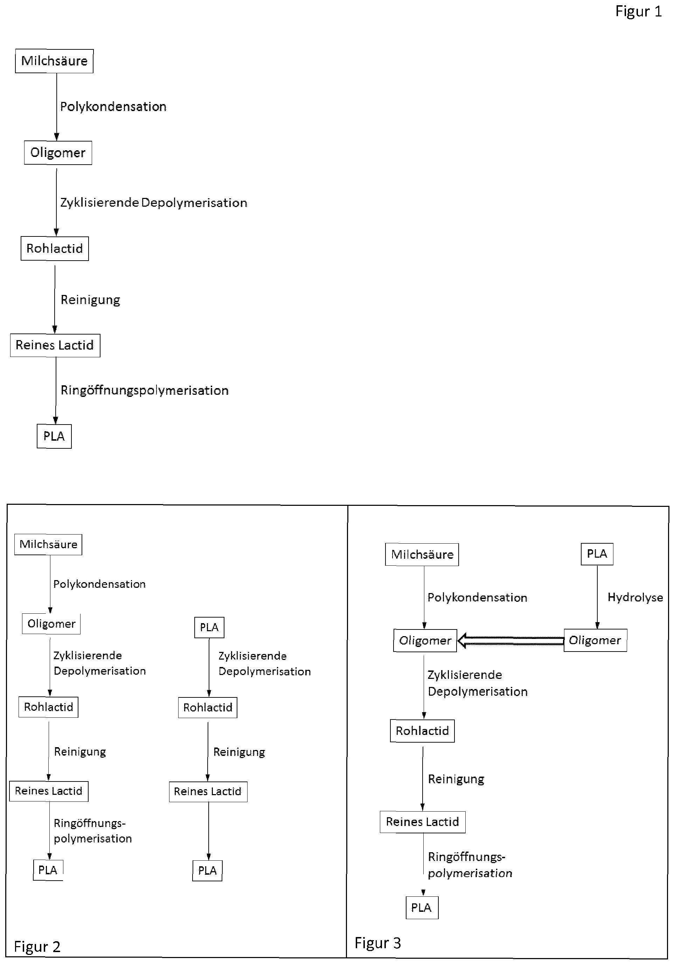

Polylactid (PLA) als prominentester Vertreter der Polyhydroxycarbonsäuren ist ein biobasierter und bioabbaubarer thermoplastischer Polyester, der technisch in einem mehrstufigen Verfahren, das schematisch in Figur 1 dargestellt ist, aus Milchsäure hergestellt wird. Der generelle Herstellungsprozess ist beispielsweise in US6277951 beschrieben. Aufgrund der Verwendung nachwachsender Rohstoffe zur Erzeugung von Milchsäure und der damit verbundenen Einsparung an fossilen, nicht erneuerbaren Rohstoffen hat PLA eine bessere CO2-Bilanz als die meisten petrochemischen Kunststoffe. Allerdings ist auch ein biobasierter Kunststoff nicht automatisch umweltfreundlich. Studien zeigen, dass PLA gegenüber beispielsweise PET in der Ökobilanz nur dann einen Vorteil besitzt, wenn das Material am Ende wiederverwertet wird. Wenn das Material nicht wiederverwertet werden kann, leidet die Nachhaltigkeit.

Zwar ist Polylactid in industriellen Kompostieranlagen bei Temperaturen über 60 °C und hoher Feuchtigkeit generell biologisch abbaubar. Allerdings wird PLA bei der Kompostierung letztendlich zu Wasser und Kohlendioxid abgebaut. Dies sind auch die Endprodukte, die bei der Verbrennung von PLA entstehen. Bei der Kompostierung erfolgt allerdings keine Energiegewinnung, das energetische Potential bleibt ungenutzt, sodass Kompostierung keine erstrebenswerte Entsorgungsstrategie für große Mengen PLA sein kann.

Daher ist es unerlässlich, effiziente und realisierbare Verwertungsstrategien für Polylactid zu entwickeln, wofür mit der Erfindungsmeldung eine Lösung angeboten wird.

Bekannte Ansätze für das Recycling von Polylactid sind das mechanische Recycling, das lösemittelbasierte Recycling und ein rohstoffliches Recycling in die Grundbausteine Milchsäure oder Dilactid (d.h. der intramolekulare Diester der Milchsäure).

Mechanisches Recycling ist für viele Kunststoffe ein etablierter Prozess. Die Kunststoffabfälle werden dabei zunächst gesammelt und sortiert, dann gewaschen und getrocknet und schließlich durch Spritzguss oder Extrusion zu neuen Produkten verarbeitet. Industrielle Abfälle wie Verschnitte, Anfahrmaterial, Angüsse werden in der Regel direkt nach dem Verarbeitungsschritt zerkleinert und der Neuware zugesetzt. Die erneute thermische Verarbeitung von Polylactid führt dabei zu einem thermo-mechanischen Abbau, der zu Veränderungen in der polymeren Struktur führt und damit auch thermische, mechanische und rheologische Eigenschaften des Kunststoffs negativ beeinflusst. Für Polyester, zu denen auch Polylactid gehört, ist die hydrolytische Kettenspaltung der Esterbindung besonders bei hohen Temperaturen ein Problem. Problematisch beim mechanischen Recycling ist außerdem die Empfindlichkeit gegenüber Verunreinigungen durch Fremdpolymere.

Für das lösemittelbasierte Recycling von PLA existiert der sogenannte CreaSolv^{®}-Prozess. In DE 10 2013 210 110 A1 wird der Prozess folgendermaßen beschrieben: Der PLA-Abfall wird in einem für PLA selektiven Lösungsmittel(-gemisch) bei Temperaturen zwischen 25 und 90°C gelöst. Nicht gelöste Bestandteile wie Additive oder Fremdpolymere werden abfiltriert. Das Polylactid wird anschließend durch Zugabe eines Fällmittels oder Temperaturabsenkung aus der Lösung ausgefällt. Dann wird das recyclierte PLA im Vakuum getrocknet bzw. im Extruder entgast und das Lösungsmittel recycelt. So können hohe Polymerreinheiten erzielt werden. Nachteil des Verfahrens ist die Notwendigkeit des Einsatzes organischer Lösemittel. Sowohl die Befreiung des PLA vom Lösemittel als auch dessen Aufbereitung sind energieintensive Prozesse.

Eine Variante des rohstofflichen Recyclings von PLA ist der vollständige Polymerabbau zu Milchsäure bzw. Milchsäureestern. Von Galactic werden dazu verschiedene Verfahrensvarianten beschrieben. Allen gemeinsam ist, dass das PLA zunächst in einem Milchsäureester gelöst und anschließend abgebaut wird. Eine Variante ist die Hydrolyse bei erhöhtem Druck und Temperaturen bis 140°C, bei der Milchsäure bzw. eines ihrer Salze entsteht, wenn die Hydrolse unter alkalischen Bedingungen durchgeführt wurde [US 2012/0142958 A1].

Alternativ werden der PLA-Lösung ein Alkohol und ein Umesterungskatalysator zugesetzt und das PLA zu Milchsäureestern abgebaut [US 2012/0029228 A1]. An den Abbauschritt schließt sich jeweils eine Reinigung der Milchsäure bzw. des Milchsäureesters durch Filtration, Destillation, Kristallisation oder andere Aufreinigungsverfahren an.

Von DuPont wird in EP0628533 der Abbau von PLA durch Wasser, Alkohole und Amine beschrieben, wobei die flüssige, Milchsäure enthaltende Phase nach dem Abbau abgetrennt und gereinigt wird. Nachteilig an diesen Verfahren ist wiederum die Verwendung von Lösungsmitteln sowie die anschließende aufwändige Reinigung der Milchsäure(ester), um eine Milchsäure in polymerisationsfähiger Qualität zu erhalten. Zudem muss die so gewonnene Milchsäure im technischen PLA-Herstellungsprozess die energieintensiven Prozesse "Entwässerung" und "Präpolykondensation" zwangsläufig durchlaufen.

Das rohstoffliche Recycling von PLA zu Dilactid, dem cyclischen Dimeren der Milchsäure, das im technischen PLA-Syntheseprozess das eigentliche Monomer darstellt, wird in einer Reihe von Patenten in verschiedenen Verfahrensvariationen beschrieben.

US 2012/0165554A1 beschreibt ein Verfahren, bei dem PLA in Milchsäureestern gelöst und zunächst unter Zusatz eines Umesterungskatalysators zu Milchsäureoligomeren abgebaut wird. Nach der Abtrennung des Lösungsmittels wird das entstandene Milchsäure-Oligomer einer cyclisierenden Depolymerisation unterworfen um Dilactid zu gewinnen. Dieses wird im Anschluss durch Kristallisation von Verunreinigungen befreit. Wiederum wird nachteiligerweise ein Lösemittel im Prozess verwendet, das abgetrennt und recycelt werden muss.

Ein Verfahren zur Herstellung von Dilactid aus PLA wird in EP0261572A1 und DE3708915A1 beschrieben. Dem Patenttext ist zu entnehmen, dass die fragliche PLA durch Entwässern von Milchsäure hergestellt wurde. Es handelt sich demnach um ein PLA-Oligomer mit Molmassen zwischen 400g/mol und 2000g/mol, wie in erstgenannter Anmeldung auch spezifiziert wird, womit diese Anmeldungen im hier betrachteten Recycling-Kontext irrelevant sind.

Hochmolekulare PLA kann bei hohen Temperaturen und reduziertem Druck thermisch zu Dilactid depolymerisiert werden. In DE19637404B4 wird eine solche direkte Depolymerisation beschrieben. Da die Depolymerisation hochmolekularer PLA aufgrund der geringeren Anzahl an OH-Gruppen langsamer abläuft als die von Milchsäure-Oligomeren, werden im beschriebenen Verfahren eine sehr hohe Katalysatorkonzentration und eine ebenfalls vergleichsweise hohe Temperatur verwendet. Dies ist nicht nur energetisch unvorteilhaft sondern erhöht auch das Ausmaß der Racemisierung, so dass Dilactid mit geringerer optischer Reinheit resultiert.

Ein ähnlicher Prozess mit hochmolekularer PLA ist in US2013/0023674A1 beschrieben. Obwohl die benötigten Depolymerisationszeiten hier nicht genannt werden, ist klar, dass der beschriebene Prozess aufgrund der geringen Endgruppenkonzentration zu langsam abläuft, um rentabel zu sein. Für lange Depolymerisationszeiten spricht auch das angegebene Ausmaß der Racemisierung.

EP1741707A1 beschreibt die Depolymerisation von PLA zu Dilactid in Gegenwart von Aluminiumhydroxid. Der Mindestgehalt von Aluminiumhydroxid in der Schmelze wird dabei mit 10wt% angegeben. Daher verbleiben im Rückstand hohe Mengen an Aluminiumoxid, die entsorgt werden müssen.

Alle vorangehend genannten Prozesse setzen ausschließlich hochmolekulare PLA im Depolymerisationsschritt ein, was das Handling hoher Schmelzviskositäten in diesem Schritt erfordert und ebenfalls unvorteilhaft ist. Dies gilt auch für WO2014/000277, die ein Verfahren beschreibt, in welchem PLA in einem Depolymerisationsreaktor zunächst unter Zusatz eines kettenspaltenden Katalysators zu einem PLA geringerer Molmasse abgebaut und anschließend unter Vakuum zu Dilactid depolymerisiert wird sowie für US2016/0311793A1, dessen Verfahren auf die erhöhte Produktion von D-Lactid bzw. meso-Lactid aus Recycling-PLA abzielt. Die genannten Verfahren folgen Recyclingstrategien, bei denen PLA-Recycling und PLA-Synthese getrennt oder parallel zueinander ohne Verbindung ablaufen (siehe hierzu das in Figur 2 dargestellte Reaktionsschema).

US2014/0316097A1 beschreibt ein rohstoffliches PLA-Recycling, bei dem PLA in einem ersten Schritt mit Wasser oder einem Milchsäure-Wasser-Gemisch zu kurzkettigen Spezies hydrolysiert wird, die dann in einem 2. Schritt cyclisierend zu Dilactid depolymerisiert werden. Dabei wird auch die Möglichkeit gezeigt, die kurzkettigen Spezies in den Depolymerisationschritt des technischen PLA-Prozesses einzuspeisen. Auch hier ist ein zusätzlicher, energieaufwändiger Schritt mit zusätzlichem apparativen Bedarf (Hydrolyse) erforderlich, um PLA zu Dilactid zu recyceln. Dieses Verfahren wird in Figur 3 veranschaulicht.

EP 1 310 496 A1 beschreibt ein Verfahren zur Herstellung eines cyclischen Esters durch Depolymerisation eines aliphatischen Polyesters. Bei diesem Verfahren wird eine Mischung, enthaltend den aliphatischen Polyester und einen spezifischen Polyalkylenglycolether, der einen Siedepunkt von 230 bis 450°C und ein Molekulargewicht von 150 bis 450°C unter Normaldruck oder reduziertem Druck auf eine Temperatur erhitzt, bei welcher die Depolymerisation des aliphatischen Polyesters stattfindet.

Ausgehend hiervon ist es Aufgabe der vorliegenden Erfindung, ein möglichst einfaches Recycling-Verfahren, das insbesondere für Polymilchsäure geeignet ist, zur Verfügung zu stellen, wobei die im Stand der Technik genannten Nachteile vermieden werden können. Insbesondere soll sich das erfindungsgemäße Verfahren apparativ einfach umsetzen lassen und hohe Ausbeuten am erhaltenen cyclischen Diester, insbesondere Dilactid, gewährleisten.

Diese Aufgabe wird mit einem Verfahren zur Herstellung eines intramolekularen Diesters einer Hydroxycarbonsäure gemäß Patentanspruch 1 gelöst. Patentanspruch 14 betrifft ein Verfahren zur Herstellung einer Polyhydroxycarbonsäure, das sich an ein Verfahren gemäß Patentanspruch 1 anschließt. Die abhängigen Patentansprüche betreffen dabei vorteilhafte Weiterbildungen.

Die vorliegende Erfindung betrifft somit ein Verfahren zur Herstellung eines intramolekularen Diesters einer Hydroxycarbonsäure, umfassend die nachfolgenden Schritte:
a) Polykondensation einer Hydroxycarbonsäure zu einer oligomeren Hydroxycarbonsäure, sowie
b) cyclisierende Depolymerisation der oligomeren Hydroxycarbonsäure um einen intramolekularen Diester der Hydroxycarbonsäure zu erhalten,
wobei
im Schritt b) ein Polyhydroxycarbonsäure-Recyclat, oder ein Kunststoffblend, enthaltend ein Polyhydroxycarbonsäure-Recyclat und zumindest einen weiteren Kunststoff zugesetzt wird. Der Begriff "Kunststoff-Recyclat" wird im Sinne der vorliegenden Erfindung insbesondere im Sinne der Norm DIN EN 15347:2007 verstanden.

Bei der vorliegenden Erfindung wird somit in die cyclisierende Depolymerisationsstufe, bei der ein intramolekularer Diester erzeugt wird, direkt ein Kunststoff-Recyclat zugegeben. Das Kunststoff-Recyclat wird dabei nicht weiter chemisch vorbehandelt, beispielsweise einem Molekulargewichtsabbau, insbesondere durch Hydrolyse, unterzogen, sondern in unbehandeltem nativen Zustand in Stufe b) des erfindungsgemäßen Verfahrens aufgegeben.

Hierzu kann beispielsweise die Polyhydroxycarbonsäure, die von der in Stufe a) eingesetzten Hydroxycarbonsäure abgeleitet ist, eingesetzt werden. Für den Fall, dass in Stufe a) beispielsweise Milchsäure eingesetzt wird, stellt die in Stufe b) im zugesetzten Kunststoff-Recyclat enthaltene Polyhydroxycarbonsäure insbesondere Polymilchsäure dar.

Es ist jedoch ebenso möglich, eine Co-Polyhydroxycarbonsäure in Stufe b) einzusetzen. Die Co-Polyhydroxycarbonsäure kann anstelle der zuvor genannten Polyhydroxycarbonsäure zugegeben werden, aber auch additiv hierzu. Die Co-Polyhydroxycarbonsäure ist dabei ebenso von der in Schritt a) eingesetzten Hydroxycarbonsäure sowie einer weiteren, sich hiervon unterscheidenden Hydroxycarbonsäure, abgeleitet. Für den Fall, dass beispielsweise in Stufe a) Milchsäure eingesetzt wird, wäre ein Beispiel einer möglichen Co-Polyhydroxycarbonsäure Polylactid-Co-Glykolid.

Das Kunststoff-Recyclat kann daneben noch Fremd-Kunststoffe, z. B. in Form eines Blends, beinhalten, die sowohl von der Polyhydroxycarbonsäure bzw. der Co-Polyhydroxycarbonsäure verschieden sind.

Mit der Direkteinspeisung von zu recycelndem Polyhydroxycarbonsäure-Recyclat (z. B. PLA-Recyclat) bzw. dieses Recyclat enthaltenden Blends in den Syntheseprozess eines Hydroxycarbonsäure-Diesters (z. B. Dilactid) und/oder einem Polymerisationsprozess dieses Diesters wird ein Recyclingverfahren ermöglicht, das nahezu keinen apparativen Mehraufwand erfordert und zu Polyhydroxycarbonsäuren führt, die von völliger Neuware ununterscheidbar sind.

Der Begriff "bestehend aus" ist im Sinne der vorliegenden Erfindung dahingehend zu verstehen, dass das Kunststoffrecyclat - für den Fall, dass es aus diesen Komponenten besteht - lediglich die genannten Komponenten beinhaltet und somit hinsichtlich des Kunststoffes oder der genannten Kunststoffe sortenrein ist. Selbstverständlich können die genannten Kunststoffe, insbesondere die Polyhydroxycarbonsäure, die Co-Polyhydroxycarbonsäure sowie ggf. der weitere Kunststoff noch die üblichen Zusatzstoffe beinhalten, die normalerweise bei der Produktion der jeweiligen Kunststoffe mit in das zur Herstellung der Kunststoffe verwendete Edukt-Gemisch eingearbeitet werden oder in einem entsprechenden Kunststoff oder einem Kunststoffblend als Zusatzstoffe zugeschlagen werden. Dies stellen insbesondere die gängigen Katalysatoren, Stabilisatoren und/oder Additive dar, die zur Einstellung des jeweiligen Eigenschaftsprofils der jeweiligen Kunststoffe herangezogen werden.

In einer bevorzugten Ausführungsform wird das Polyhydroxycarbonsäure-Recyclat als Schmelze, z.B. mittels eines Extruders zugesetzt. Gemäß dieser bevorzugten Ausführungsform wird das Kunststoff-Recyclat direkt aufgeschmolzen und in Stufe b) gegeben.

Das Kunststoff-Recyclat wird hierbei normalerweise in Form von Chips oder als Granulat erhalten und kann somit direkt in dieser Form dem Extruder zugegeben und aufgeschmolzen werden.

Ebenso ist es jedoch möglich, das Kunststoff-Recyclat, insbesondere in Form von Chips oder Granulat direkt, d. h. ohne Aufschmelzen, in die Stufe b) aufzugeben.

Gemäß einer weiteren bevorzugten Ausführungsform ist vorgesehen, dass das Polyhydroxycarbonsäure-Recyclat in Schritt b), bezogen auf die oligomere Hydroxycarbonsäure, zu bis zu maximal 50 Gew.-%, bevorzugt 0,01 bis 40 Gew.-%, weiter bevorzugt 0,1 bis 30 Gew.-%, weiter bevorzugt 1 bis 25 Gew.-%, weiter bevorzugt 2,5 bis 20 Gew.-%, besonders bevorzugt 5 bis 15 Gew.-% zugesetzt wird.

Die cyclisierende Depolymerisation, die im Schritt b) durchgeführt wird, erfolgt bevorzugt bei Temperaturen zwischen 200 und 250 °C, besonders bevorzugt zwischen 210 und 230 °C.

Das Kunststoff-Recyclat besteht in einer bevorzugten Ausführungsform aus genau einer Polyhydroxycarbonsäure bzw. einer Co-Polyhydroxy-carbonsäure. Insbesondere ist bevorzugt, dass das Kunststoff-Recyclat aus einer Polyhydroxycarbonsäure, ganz besonders bevorzugt Polymilchsäure (bzw. synonym Polylactid) besteht.

Jedoch ist es ebenso möglich, dass das Kunststoff-Recyclat einen Blend, enthaltend oder bestehend aus mindestens einer Polyhydroxycarbonsäure und/oder mindestens einer Co-Polyhydroxycarbonsäure sowie mindestens einen von der mindestens einen Polyhydroxycarbonsäure und der mindestens einen Co-Polyhydroxycarbonsäure verschiedenen Kunststoff, beinhaltet oder hieraus besteht.

Des Weiteren ist es möglich, dass das Kunststoff-Recyclat einen Blend, enthaltend oder bestehend aus mindestens zwei verschiedenen Polyhydroxycarbonsäuren oder mindestens zwei verschiedenen Co-Polyhydroxycabonsäuren, beinhaltet oder hieraus besteht.

Für den Fall, dass der Blend einem von der mindestens einen Polyhydroxycarbonsäure und der mindestens einen Co-Polyhydroxycarbonsäure verschiedenen Kunststoff beinhaltet, ist es bevorzugt, wenn dieser ausgewählt ist aus der Gruppe bestehend aus Polyestern, insbesondere Polybutylensuccinat (PBS), Polybutylenadipat-co-terephthalat (PBAT, Ecoflex^{®}), Polybutylensuccinat-co-adipat, Polyhydroxyalkanoaten (PHA), Polyethylenterephthalat (PET), Polybutylenterephthalat (PBT), Polytrimethylenterephthalat (PTT); Polyamiden; Polyvinylacetat; Polyolefinen, insbesondere Polyethylen (PE) und/oder Polypropylen (PP); Ethylen-Vinylacetat-Copolymeren; Polycarbonaten; Polyvinylalkohol (PVA); Polyethern, insbesondere Polyethylenglykol (PEG), Polytrimethylenglykol (PTMG), Polypropylenglykol (PPG) oder Copolymeren davon; Polystyrol; Acrylnitril-Butadien-Styrol-Copolymeren; Polyacetaten; Poly(meth)acrylaten; sowie thermoplastischen und duroplastischen Elastomeren.

Dieser weitere Kunststoff fällt normalerweise in Stufe b) als Sumpfbestandteil an und kann ggf. aus Stufe b) zusammen mit dem Sumpf abgetrennt werden.

Insbesondere wird der in Schritt b) erhaltene intramolekulare Diester der Hydroxycarbonsäure im gasförmigen Aggregatszustand abgetrennt. Dies ermöglicht insbesondere den Betrieb des erfindungsgemäßen Verfahrens in kontinuierlicher Weise. Selbstverständlich ist es ebenso möglich, das erfindungsgemäße Verfahren batchweise durchzuführen.

Für den Fall, dass das Kunststoffrecyclat eine oder mehrere Polyhydroxycarbonsäuren (bzw. deren Co-Polymere) beinhaltet, die nicht von der in Schritt a) eingesetzten Hydroxycarbonsäure abgeleitet sind (bzw. im Falle von Co-Polymeren diese beinhalten) wird in der Stufe b) ein Gemisch gebildet, das den intramolekularen Diester der in Schritt a) eingesetzten Hydroxycarbonsäure beinhaltet, jedoch noch weitere Diester, die von der bzw. den weiteren Hydroxycarbonsäuren abgeleitet sind.

In diesem Fall ist es bevorzugt, den intramolekularen Diester der in Schritt a) eingesetzten Hydroxycarbonsäure aus diesem Gemisch abzutrennen, beispielsweise durch Destillation bzw. Rektifikation.

Im Detail ist es somit bevorzugt, dass für den Fall, dass das in Schritt b) zugesetzte Kunststoff-Recyclat zusätzlich z. B.

mindestens eine Polyhydroxycarbonsäure, die nicht von der in Schritt a) eingesetzten Hydroxycarbonsäure abgeleitet ist,
mindestens eine Co-Polyhydroxycarbonsäure, wobei mindestens eine Co-Polyhydroxycarbonsäure von der in Schritt a) eingesetzten Hydroxycarbonsäure sowie mindestens einer von der in Schritt a) eingesetzten Hydroxycarbonsäure verschiedenen Hydroxycarbonsäure abgeleitet ist, und/oder
zusätzlich mindestens eine Co-Polyhydroxycarbonsäure, wobei mindestens eine Co-Polyhydroxycarbonsäure ausschließlich von mindestens zwei von der in Schritt a) eingesetzten Hydroxycarbonsäure verschiedenen Hydroxycarbonsäuren abgeleitet ist,
enthält, ein Gemisch, enthaltend den intramolekularen Diester der in Schritt a) eingesetzten Hydroxycarbonsäure, einen intramolekularen Diester der in Schritt a) eingesetzten Hydroxycarbonsäure und einer von der in Schritt a) eingesetzten Hydroxycarbonsäure verschiedenen Hydroxycarbonsäure sowie einen intramolekularen Diester aus mindestens einer von der in Schritt a) eingesetzten Hydroxycarbonsäure verschiedenen Hydroxycarbonsäure, erhalten wird, und
der intramolekularen Diester der in Schritt a) eingesetzten Hydroxycarbonsäure aus dem Gemisch abgetrennt wird, bevorzugt mittels Destillation.

Beim erfindungsgemäßen Verfahren ist es ebenso möglich, dass bei der zuvor beschriebenen Abtrennung auch die weiteren intramolekularen Diester (insbesondere der intramolekulare Diester der in Schritt a) eingesetzten Hydroxycarbonsäure und einer von der in Schritt a) eingesetzten Hydroxycarbonsäure verschiedenen Hydroxycarbonsäure und/oder der intramolekulare Diester aus einer von der in Schritt a) eingesetzten Hydroxycabronsäure verschiedenen Hydroxycarbonsäure) zusätzlich abgetrennt und als Reinsubstanz erhalten werden können.. Die Auftrennung der verschiedenen Diester kann z. B. mittels Destillation und/oder Umkristallisation erfolgen. Die erhaltenen Diester können zusätzlich in einer weiteren Stufe aufgereinigt werden.

Weiter vorteilhaft kann in Schritt b) zusätzlich ein Alkohol mit einem bei Standardbedingungen (definiert gemäß IUPAC, 1982, d. h. bei einem Druck von 10⁵ Pa) bestimmten Siedepunkt von ≥ 150 °C zugesetzt werden, bevorzugt in einer Menge von 0,01 bis 2 Gew.-%, besonders bevorzugt von 0,1 bis 0,5 Gew.-%, bezogen auf die Gesamtmenge der in Schritt b) eingesetzten Verbindungen.

Dieser Alkohol kann insbesondere ausgewählt sein aus der Gruppe bestehend aus Alkandiolen, insbesondere Ethylenglycol, Propylenglycol, und/oder Butylenglycol; Fettalkoholen mit 6 bis 22 Kohlenstoffatomen, insbesondere Decanol; Alkantriolen, cyclischen Alkoholen sowie Mischungen und Kombinationen hiervon.

Die in Schritt a) eingesetzte Hydroxycarbonsäure (aus der sich auch die Polyhydroxycarbonsäure und/oder Co-Polyhydroxycarbonsäure, die im Kunststoff-Recyclat enthalten sind, ableiten, ist hierbei insbesondere ausgewählt aus der Gruppe bestehend aus 2-Hydroxypropansäure, 2-Hydroxyethansäure, 2-Hydroxybutansäure, 2-Hydroxypentansäure, 2-Hydroxyhexansäure, 2-Hydroxyheptansäure, 2-Hydroxyoctansäure, 2-Hydroxy-2-methylpropansäure, 2-Hydroxy-2-methylbutansäure, 2-Hydroxy-2-ethylbutansäure, 2-Hydroxy-2-methylpentansäure, 2-Hydroxy-2-ethylpentansäure, 2-Hydroxy-2-propyl-pentansäure, 2-Hydroxy-2-butylpentansäure, 2-Hydroxy-2-methylhexansäure, 2-Hydroxy-2-ethylhexansäure, 2-Hydroxy-2-propylhexansäure, 2-Hydroxy-2-butylhexansäure, 2-Hydroxy-2-pentylhexansäure, 2-Hydroxy-2-methylheptansäure, 2-Hydroxy-2-ethylheptansäure, 2-Hydroxy-2-propylheptansäure, 2-Hydroxy-2-butylheptansäure, 2-Hydroxy-2-pentylheptansäure, 2-Hydroxy-2-hexylheptansäure, 2-Hydroxy -2-Methyloctansäure, 2-Hydroxy-2-ethyloctansäure, 2-Hydroxy-2-propyloctansäure, 2-Hydroxy-2-butyloctansäure, 2-Hydroxy-2-pentyloctansäure, 2-Hydroxy-2-hexyloctansäure, 2-Hydroxy-2-heptyloctansäure, 3-Hydroxypropansäure, 3-Hydroxybutansäure, 3-Hydroxypentansäure, 3-Hydroxyhexansäure, 3-Hydroxyheptansäure, 3-Hydroxyoctansäure, 3-Hydroxy-3-methylbutansäure, 3-Hydroxy-3-methylpentansäure, 3-Hydroxy-3-ethylpentansäure, 3-Hydroxy-3-methylhexansäure, 3-Hydroxy-3-ethylhexansäure, 3-Hydroxy-3-propylhexansäure, 3-Hydroxy-3-methylheptansäure, 3-Hydroxy-3-ethylheptansäure, 3-Hydroxy-3-propylheptansäure, 3-Hydroxy-3-butylheptansäure, 3-Hydroxy-3-methyloctansäure, 3-Hydroxy-3-ethyloctansäure, 3-Hydroxy-3-propyloctansäure, 3-Hydroxy-3-pentyloctansäure, 4-Hydroxybutansäure, 4-Hydroxypentansäure, 4-Hydroxyhexansäure, 4-Hydroxyheptansäure, 4-Hydroxyoctansäure, 4-Hydroxy-4-methylpentansäure, 4-Hydroxy-4-Methylhexansäure, 4-Hydroxy-4-ethylhexansäure, 4-Hydroxy-4-metylheptansäure, 4-Hydroxy-4-ethylheptansäure, 4-Hydroxy-4-propylheptansäure, 4-Hydroxy-4-methyloctansäure, 4-hydroxy-4-Ethyloctansäure, 4-Hydroxy-4-propyloctansäure, 4-Hydroxy-4-butyloctansäure, 5-Hydroxypentansäure, 5-Hydroxyhexansäure, 5-Hydroxyheptansäure, 5-Hydroxyoctansäure, 5-Hydroxy-5-methylhexansäure, 5-Hydroxy-5-methylheptansäure, 5-Hydroxy-5-ethylheptansäure, 5-Hydroxy-5-methyloctansäure, 5-Hydroxy-5-ethyloctansäure, 5-Hydroxy-5-propyloctansäure, 6-Hydroxyhexansäure, 6-Hydroxyheptansäure, 6-Hydroxyoctansäure, 6-Hydroxy-6-methylheptansäure, 6-Hydroxy-6-methyloctansäure, 6-Hydroxy-6-ethyloctansäure, 7-Hydroxyheptansäure, 7-Hydroxyoctansäure, 7-Hydroxy-7-methyloctansäure und 8-Hydroxyoctansäure.

Besonders bevorzugt ist hierbei 2-Hydroxypropansäure, d. h. Milchsäure. In einer besonders bevorzugten Ausführungsform betrifft die vorliegende Erfindung somit ein Verfahren zur Herstellung von Dilactid, umfassend die
a) Polykondensation von Milchsäure zu einem Milchsäureoligomer, sowie
b) cyclisierende Depolymerisation des Milchsäureoligomers zu Lactid, wobei in Schritt b) ein Kunststoffrecyclat, enthaltend oder bestehend aus Polymilchsäure (bzw. Polylactid) zugesetzt wird.

Weiter ist es vorteilhaft, dass nach Schritt b) eine Aufreinigung des erhaltenen intramolekularen Diesters der in Schritt a) eingesetzten Hydroxycarbonsäure erfolgt, z.B. durch Destillation und/oder Kristallisation.

Zudem betrifft die vorliegende Erfindung ein Verfahren zur Herstellung einer Polyhydroxycarbonsäure, beispielsweise Polymilchsäure, bei dem im An schluss an ein im Vorangehenden beschriebenes Verfahren zur Herstellung eines intramolekularen Diesters der erhaltene intramolekulare Diester, z. B. Dilactid, in einer ringöffnenden Polymerisation eingesetzt wird.

Die vorliegende Erfindung wird nachfolgend anhand des Beispiels des Recyclings von Polylactid (d. h. Polymilchsäure) in die Stufe der cyclisierenden Depolymerisation eines Milchsäureoligomers näher ausgeführt, ohne die vorliegende Erfindung auf die dargestellte Ausführungsform zu beschränken.

Die Erfindung beschreibt anders als die eingangs genannten Verfahren einen Weg, z. B. hochmolekulares, zu recycelndes PLA direkt in den Depolymerisationsschritt des technischen PLA-Prozesses einzuspeisen, ohne apparative oder prozesstechnische Änderungen vornehmen zu müssen.

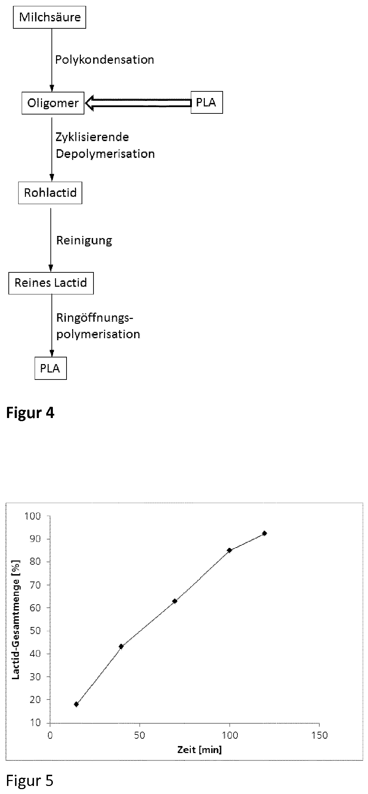

Eine Möglichkeit zur Ausführung der Erfindung ist schematisch in Fig. 4 dargestellt. Das zu recycelnde Polylactid wird hierbei in die zweite dargestellte Stufe aufgegeben, bei der eine cyclisierende Depolymerisation des in der Stufe vorliegenden Oligomeren (Milchsäureoligomere) erfolgt.

Kern der vorliegenden Erfindung ist, dass das zu recycelnde PLA gemeinsam mit dem Milchsäure-Oligomer der cyclisierenden Depolymerisation unterworfen wird. Technisch kann dies beispielsweise durch eine Dosierung des zu recycelnden PLA als Schmelze mit Hilfe eines Extruders direkt in den Depolymerisationskessel erfolgen. Die Depolymerisationstemperaturen liegen dabei zwischen 200°C und 250°C, idealerweise zwischen 210°C und 230°C. Mögliche Anteile des PLAs an der Gesamtmasse des Depolymerisationsschrittes liegen bei maximal 50%, idealerweise bei 5-15%. Da im Depolymerisationskessel sehr schnell eine Umesterung zwischen den Oligomeren und der hochmolekularen PLA stattfindet, verringert sich die Geschwindigkeit der Depolymerisation zu Dilactid nicht und es sind auch keine höheren Temperaturen oder Katalysatormengen erforderlich. (Beispiele 1 und 2→ Ingeo-Granulate, Vergleichsbeispiel 1 → Oligomer).

Der gefundene Recyclingprozess kann nicht nur auf reines PLA angewendet werden, sondern auch auf Blends von PLA mit anderen Polymeren (Polyestern, Polyamiden, Polyvinylacetat etc.). Für viele Blendkomponenten ist dies ohne Prozessanpassung möglich. Die Blendkomponente greift nicht in die Depolymerisationsreaktion ein und verbleibt nach der Depolymerisation des PLA im Sumpf des Depolymerisationskessels. (Beispiel 3 → PBS-Compound) Im Falle von Blendkomponenten, die die Geschwindigkeit der Depolymerisation reduzieren, ist der Zusatz eines Alkohols, idealerweise eines relativ hochsiedenden Alkohols wie Ethylenglykol, Propandiol, Butandiol, Decanol etc. in einer Menge von 0,01-2wt%, idealerweise zwischen 0,1 und 0,5 wt% , vorteilhaft. Ohne Zusatz dieses Alkohols verläuft die Depolymerisation zu langsam, um in den technischen Prozess integriert werden zu können (Vergleichsbeispiel 2). Dies kann über eine Erhöhung der Depolymerisationstemperatur aufgefangen werden, was aber zu unerwünschter verstärkter Racemisierung mit meso-Lactid-Gehalten >6% führt. Bei Zusatz des Alkohols verläuft die Depolymerisation wieder störungsfrei und mit geringerer Racemisierung (Beispiel 4). Geschwindigkeitsreduzierende Blendkomponenten sind Polymere oder niedermolekulare Substanzen, die beispielsweise über Umesterungsreaktionen die OH-Endgruppen des PLA blockieren. Genannt seien beispielsweise Polyvinylacetat (Bestandteil von Vinnex, eines häufig genutzten Blendpartners für PLA) und Polyvinylalkohol.

Natürlich kann der Alkohol vorsichtshalber unabhängig von evtl. Blendkomponenten eingesetzt werden, so dass nicht in Abhängigkeit von der Qualität des zu recycelnden PLA Prozessanpassungen erforderlich sind.

### Synthese des PLA-Oligomeren für die Depolymerisationsversuche

Für die Synthese des Präkondensates (PLA-Oligomer) wurden in einem emaillierten Metallreaktor (25 L) mit Glasdeckel und Propellerrührer 20,03 kg L-Milchsäure (ω = 90%) (200 mol) und 16,67 g Butylzinnhydroxid-oxid (80 mmol) als Katalysator dispergiert und auf 120 °C erhitzt. Der Druck wurde auf 400 mbar reduziert, um die Entwässerung und Kondensation der Milchsäure zu starten. Der weitere Verlauf des Druck- und Temperaturprogramms ist der Tabelle zu entnehmen:

| Zeit [min] | Druck [mbar] | Öltemperatur [°C] |
|---|---|---|
| 0 | 400 | 120 |
| 30 | 300 | 130 |
| 45 | 200 | 130 |
| 60 | 200 | 140 |
| 120 | 200 | 150 |
| 135 | 200 | 160 |
| 150 | 200 | 170 |
| 165 | 200 | 180 |
| 255 | 150 | 180 |
| 270 | 100 | 180 |
| 285 | 50 | 180 |
| 360 | Versuchsende | |

Nach der Reaktionszeit von 6 Stunden wurde das PLA-Oligomer aus dem Reaktor ausgefahren und bis zur weiteren Verwendung im Kühlschrank aufbewahrt.
GPC (in Dichlormethan gegen Polystyrol): Mn = 700 g/mol; PD = 1,71
HPLC: 0,84% D-Milchsäure

### Genereller Ablauf der Depolymerisationsversuche

Die Depolymerisation wurde in einem 1L-Büchi-Metallreaktor durchgeführt. Sowohl der Reaktor als auch der Reaktordeckel waren temperierbar. Der Reaktor war mit einem Ankerrührer, einem Innentemperaturfühler, einer Stickstoffzufuhr und einem Drucksensor, der mit dem Vakuumpumpenstand verbunden war, ausgestattet. Die mit dem Reaktor verbundene Destillationskolonne und der sich daran anschließende Kühler waren ebenfalls temperierbar. Zwischen Destillationskolonne und Kühler bzw. am Ausgang des Kühlers befand sich jeweils ein Kugelhahn, der es gestattete, nur Teile der Apparatur zu belüften. Beide Hähne waren mit einem Heizband umwickelt, um ein Erstarren des Lactids in diesem Bereich zu vermeiden.

Der Vorlagekolben wurde mit dem Ausgang des Kühlers verbunden. Der Vakuumpumpenstand war über einen Schlauch an den Vorlagekolben angeschlossen. Zwischen Vorlagekolben und Pumpenstand befand sich eine Kühlfalle mit flüssigem Stickstoff.

Vor Beginn des Versuches wurde der Reaktormantel auf 100 °C temperiert, die Temperatur der Destillationskolonne und des Kühlers wurde auf 105 °C eingestellt. PLA wurde in der gewünschten Menge in den Reaktor dosiert. Anschließend wurde das Präpolymer aufgeschmolzen und in der gewünschten Menge mit Hilfe eines temperierten Metalltrichters in den Reaktor gefüllt.

Sobald sich alle Komponenten im Reaktor befanden, wurde die Temperatur des Reaktormantels auf die Reaktionstemperatur eingestellt und der Druck im System auf 100 mbar reduziert. Der Rührer wurde auf eine Drehzahl von ^{~} 85 min-1 eingestellt. Sobald der erste Tropfen Destillat übertrat, begann die Kondensationsphase (45-50 min). In dieser Zeit wurde noch kein Dilactid destilliert. Das während dieser Zeit gewonnene Destillat war flüssig und wurde als Vorlauf verworfen. Erst danach begann mit der Absenkung des Druckes auf 10 mbar die eigentliche Dilactiddestillation (Destillationsphase ab 50 min). Das weitere Druckprogramm für den Reaktionsverlauf ist in der nachfolgenden Tabelle zusammengefasst.

| Zeit [min] | Druck [mbar] | Bemerkungen |
|---|---|---|
| 0 | 100 | Start des Versuches bei erstem Destillat |
| 30 | 50 | |
| 50 | 10 | |
| 120 | 5 | |
| 170 | 5 | Versuchsende |

Vergleichsbeispiel 1 (nicht erfindungsgemäß, da nur Oligomer im Depolymerisationsschritt verwendet):
Die Depolymerisation wurde bei 225°C entsprechend dem oben beschriebenen generellen Ablauf durchgeführt. Es wurden 298,96g Präkondensat im Versuch eingesetzt, die Lactidausbeute betrug 276,55g (92,5%). Der Anteil an meso-Lactid lag bei 2,9%. Den Verlauf der Reaktion zeigt Figur 5.

### Beispiel 1 (erfindungsgemäß)

In den Depolymerisationsreaktor wurden 29,80g Ingeo^{™} 3251D-Granulat (PLA-Spritzgusstype von NatureWorks) und 268,71g Präpolykondensat eingewogen. Der Anteil an PLA betrug damit 10wt%. Die Depolymerisation wurde bei 225°C entsprechend dem oben beschriebenen generellen Ablauf durchgeführt. Die Ausbeute an Dilactid betrug 273,00g (91,5%), der Anteil an meso-Lactid 3,06%.

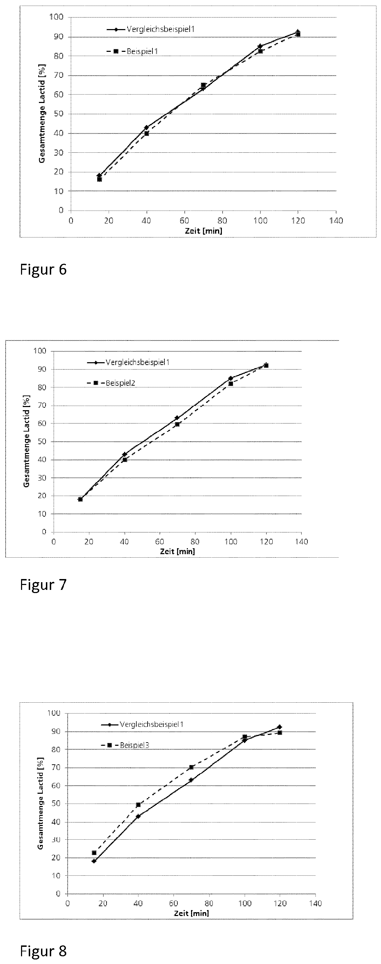

Den Verlauf der Reaktion im Vergleich zu Vergleichsbeispiel 1 zeigt Figur 6.

### Beispiel 2 (erfindungsgemäß)

In den Depolymerisationsreaktor wurden 30,03g Ingeo^{™} 7000D-Granulat (PLA-Blasform von NatureWorks) und 267,71g Präpolykondensat eingewogen. Der Anteil an PLA betrug damit 10wt%. Die Depolymerisation wurde bei 225°C entsprechend dem oben beschriebenen generellen Ablauf durchgeführt. Die Ausbeute an Dilactid betrug 274,41g (92,2%), der Anteil an meso-Lactid 3,11%.

Den Verlauf der Reaktion im Vergleich zu Vergleichsbeispiel 1 zeigt Figur7.

### Beispiel 3 (erfindungsgemäß)

In den Depolymerisationsreaktor wurden 30,06g eines 1:1 Blends aus Ingeo^{™} 2003D (PLA-Folientype von NatureWorks) und FZ71PL (Polybutylensuccinat-Type von Mitsubishi) und 274,95g Präpolykondensat eingewogen. Der Anteil des Blends an der Gesamtmasse betrug damit 10wt%, der PLA-Anteil 5wt%. Die Depolymerisation wurde bei 225°C entsprechend dem oben beschriebenen generellen Ablauf durchgeführt. Die Ausbeute an Dilactid betrug 260,57g (89,9%), der Anteil an meso-Lactid 4,16%.

Den Verlauf der Reaktion im Vergleich zu Vergleichsbeispiel 1 zeigt Figur 8.

Vergleichsbeispiel 2 (nicht erfindungsgemäß, da für Recycling eines Blends mit geschwindigkeitsreduzierender Blendkomponente kein Alkoholzusatz):
In den Depolymerisationsreaktor wurden 30,10g eines Materials, das aus gewaschenen und zerkleinerten Danone-Joghurtbechern (Blend aus Ingeo^{™} 2003D, einer Vinnex-Type von Wacker, Titandioxid und ggf. weiteren Additiven in unbekannter Menge) gewonnen wurde, und 270,89g Präpolykondensat eingewogen. Der Anteil an PLA-Material betrug damit 10wt%, der Anteil an reinem PLA ist aufgrund der unbekannten Zusammensetzung des Joghurtbechers nicht bekannt, wird aber auf 8,5% geschätzt. Die Depolymerisation wurde bei 225°C entsprechend dem oben beschriebenen generellen Ablauf durchgeführt. Die Ausbeute an Dilactid betrug 246,16g (81,8%), der Anteil an meso-Lactid 4,8%.

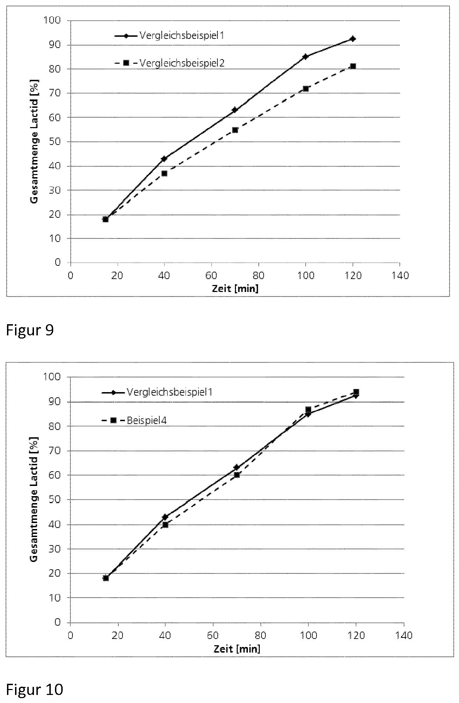

Den Verlauf der Reaktion im Vergleich zu Vergleichsbeispiel 1 zeigt Figur 9.

**Beispiel 4 (erfindungsgemäß, Recycling eines Blends** mit geschwindigkeitsreduzierender Blendkomponente **unter Alkoholzusatz):**
In den Depolymerisationsreaktor wurden 30,49g eines Materials, das aus gewaschenen und zerkleinerten Danone-Joghurtbechern (Blend aus Ingeo^{™} 2003D, einer Vinnex-Type von Wacker, Titandioxid und ggf. weiteren Additiven in unbekannter Menge) gewonnen wurde, und 272,98g Präpolykondensat eingewogen. Zusätzlich wurden 0,48g Ethylenglykol (0,16wt%) zugefügt. Der Anteil an PLA-Material betrug damit 10wt%, der Anteil an reinem PLA ist aufgrund der unbekannten Zusammensetzung des Joghurtbechers nicht bekannt, wird aber auf 8,5% geschätzt. Die Depolymerisation wurde bei 225°C entsprechend dem oben beschriebenen generellen Ablauf durchgeführt. Die Ausbeute an Dilactid betrug 286,92g (94,57%), der Anteil an meso-Lactid 5,4%.

Den Verlauf der Reaktion im Vergleich zu Vergleichsbeispiel 1 zeigt Figur 10.

## Patentansprüche

1. Verfahren zur Herstellung eines intramolekularen Diesters einer Hydroxycarbonsäure, umfassend die nachfolgenden Schritte:
a) Polykondensation einer Hydroxycarbonsäure zu einer oligomeren Hydroxycarbonsäure, sowie
b) cyclisierende Depolymerisation der oligomeren Hydroxycarbonsäure um einen intramolekularen Diester der in Schritt a) eingesetzten Hydroxycarbonsäure zu erhalten,
**dadurch gekennzeichnet, dass**
im Schritt b) ein Kunststoff-Recyclat, enthaltend oder bestehend aus mindestens einer Polyhydroxycarbonsäure, wobei mindestens eine Polyhydroxycarbonsäure von der in Schritt a) eingesetzten Hydroxycarbonsäure abgeleitet ist, und/oder
mindestens einer Co-Polyhydroxycarbonsäure, wobei mindestens eine Co-Polyhydroxycarbonsäure von der in Schritt a) eingesetzten Hydroxycarbonsäure sowie mindestens einer von der in Schritt a) eingesetzten Hydroxycarbonsäure verschiedenen Hydroxycarbonsäure abgeleitet ist,
zugesetzt wird.

2. Verfahren nach Anspruch 1, **dadurch gekennzeichnet, dass** das Kunststoff-Recyclat als Schmelze, z.B. mittels eines Extruders zugesetzt wird.

3. Verfahren nach einem der vorhergehenden Ansprüche, **dadurch gekennzeichnet, dass** das Kunststoff-Recyclat in Schritt b), bezogen auf die oligomere Hydroxycarbonsäure, zu bis zu maximal 50 Gew.-%, bevorzugt 0,01 bis 40 Gew.-%, weiter bevorzugt 0,1 bis 30 Gew.-%, weiter bevorzugt 1 bis 25 Gew.-%, weiter bevorzugt 2,5 bis 20 Gew.-%, besonders bevorzugt 5 bis 15 Gew.-% zugesetzt wird.

4. Verfahren nach einem der vorhergehenden Ansprüche, **dadurch gekennzeichnet, dass** der Schritt der cyclisierenden Depolymerisation b) bei Temperaturen zwischen 200 und 250 °C, bevorzugt zwischen 210 und 230 °C durchgeführt wird.

5. Verfahren nach einem der vorhergehenden Ansprüche, **dadurch gekennzeichnet, dass** das Kunststoff-Recyclat
eine Polyhydroxycarbonsäure oder eine Co-Polyhydroxycabonsäure,
einen Blend, enthaltend oder bestehend aus mindestens einer Polyhydroxycarbonsäure und/oder mindestens einer Co-Polyhydroxy-carbonsäure sowie mindestens einen von der mindestens einen Polyhydroxycarbonsäure und der mindestens einen Co-Polyhydroxy-carbonsäure verschiedenen Kunststoff, oder
einen Blend, enthaltend oder bestehend aus mindestens zwei verschiedenen Polyhydroxycarbonsäuren oder mindestens zwei verschiedenen Co-Polyhydroxycabonsäuren,
beinhaltet oder hieraus besteht.

6. Verfahren nach einem der beiden vorhergehenden Ansprüche, **dadurch gekennzeichnet, dass** der mindestens eine von der mindestens einen Polyhydroxycarbonsäure und der mindestens einen Co-Polyhydroxycarbonsäure verschiedene Kunststoff ausgewählt ist aus der Gruppe bestehend aus Polyestern, insbesondere Polybutylensuccinat (PBS), Polybutylenadipat-co-terephthalat (PBAT, Ecoflex^{®}), Polybutylensuccinat-co-adipat, Polyhydroxyalkanoaten (PHA), Polyethylenterephthalat (PET), Polybutylenterephthalat (PBT), Polytrimethylenterephthalat (PTT); Polyamiden; Polyvinylacetat; Polyolefinen, insbesondere Polyethylen (PE) und/oder Polypropylen (PP); Ethylen-Vinylacetat-Copolymeren; Polycarbonaten; Polyvinylalkohol (PVA); Polyethern, insbesondere Polyethylenglykol (PEG), Polytrimethylenglykol (PTMG), Polypropylenglykol (PPG) oder Copolymeren davon; Polystyrol; Acrylnitril-Butadien-Styrol-Copolymeren; Polyacetaten; Poly(meth)acrylaten; sowie thermoplastischen und duroplastischen Elastomeren.

7. Verfahren nach einem der vorhergehenden Ansprüche, **dadurch gekennzeichnet, dass** der mindestens eine weitere Kunststoff als Sumpfbestandteil anfällt.

8. Verfahren nach einem der vorhergehenden Ansprüche, **dadurch gekennzeichnet, dass** der in Schritt b) erhaltene intramolekulare Diester der Hydroxycarbonsäure im gasförmigen Aggregatszustand abgetrennt wird.

9. Verfahren nach einem der vorhergehenden Ansprüche, **dadurch gekennzeichnet, dass** für den Fall, dass das in Schritt b) zugesetzte Kunststoff-Recyclat
zusätzlich mindestens eine Polyhydroxycarbonsäure, die nicht von der in Schritt a) eingesetzten Hydroxycarbonsäure abgeleitet ist,
mindestens eine Co-Polyhydroxycarbonsäure, wobei mindestens eine Co-Polyhydroxycarbonsäure von der in Schritt a) eingesetzten Hydroxycarbonsäure sowie mindestens einer von der in Schritt a) eingesetzten Hydroxycarbonsäure verschiedenen Hydroxycarbonsäure abgeleitet ist, und/oder
zusätzlich mindestens eine Co-Polyhydroxycarbonsäure, wobei mindestens eine Co-Polyhydroxycarbonsäure ausschließlich von mindestens zwei von der in Schritt a) eingesetzten Hydroxycarbonsäure verschiedenen Hydroxycarbonsäuren abgeleitet ist,
enthält,
ein Gemisch, enthaltend den intramolekularen Diester der in Schritt a) eingesetzten Hydroxycarbonsäure, einen intramolekularen Diester der in Schritt a) eingesetzten Hydroxycarbonsäure und einer von der in Schritt a) eingesetzten Hydroxycarbonsäure verschiedenen Hydroxycarbonsäure sowie einen intramolekularen Diester aus mindestens einer von der in Schritt a) eingesetzten Hydroxycarbonsäure verschiedenen Hydroxycarbonsäure, erhalten wird, und
der intramolekularen Diester der in Schritt a) eingesetzten Hydroxycarbonsäure aus dem Gemisch abgetrennt wird, bevorzugt mittels Destillation.

10. Verfahren nach einem der vorhergehenden Ansprüche, **dadurch gekennzeichnet, dass** in Schritt b) zusätzlich ein Alkohol mit einem bei Standardbedingungen (IUPAC, 1982) bestimmten Siedepunkt von ≥ 150 °C zugesetzt wird, bevorzugt in einer Menge von 0,01 bis 2 Gew.-%, besonders bevorzugt von 0,1 bis 0,5 Gew.-%, bezogen auf die Gesamtmenge der in Schritt b) eingesetzten Verbindungen.

11. Verfahren nach vorhergehendem Anspruch, **dadurch gekennzeichnet, dass** der Alkohol ausgewählt ist aus der Gruppe bestehend aus Alkandiolen, insbesondere Ethylenglycol, Propylenglycol, und/oder Butylenglycol; Fettalkoholen mit 6 bis 22 Kohlenstoffatomen, insbesondere Decanol; Alkantriolen, cyclischen Alkoholen sowie Mischungen und Kombinationen hiervon.

12. Verfahren nach einem der vorhergehenden Ansprüche, **dadurch gekennzeichnet, dass** die in Schritt a) eingesetzte Hydroxycarbonsäure ausgewählt ist aus der Gruppe bestehend aus 2-Hydroxypropansäure, 2-Hydroxyethansäure, 2-Hydroxybutansäure, 2-Hydroxypentansäure, 2-Hydroxyhexansäure, 2-Hydroxyheptansäure, 2-Hydroxyoctansäure, 2-Hydroxy-2-methylpropansäure, 2-Hydroxy-2-methylbutansäure, 2-Hydroxy-2-ethylbutansäure, 2-Hydroxy-2-methylpentansäure, 2-Hydroxy-2-ethylpentansäure, 2-Hydroxy-2-propylpentansäure, 2-Hydroxy-2-butylpentansäure, 2-Hydroxy-2-methylhexansäure, 2-Hydroxy-2-ethylhexansäure, 2-Hydroxy-2-propylhexansäure, 2-Hydroxy-2-butylhexansäure, 2-Hydroxy-2-pentylhexansäure, 2-Hydroxy-2-methylheptansäure, 2-Hydroxy-2-ethylheptansäure, 2-Hydroxy-2-propylheptansäure, 2-Hydroxy-2-butylheptansäure, 2-Hydroxy-2-pentylheptansäure, 2-Hydroxy-2-hexylheptansäure, 2-Hydroxy-2-Methyloctansäure, 2-Hydroxy-2-ethyloctansäure, 2-Hydroxy-2-propyloctansäure, 2-Hydroxy-2-butyloctansäure, 2-Hydroxy-2-pentyloctansäure, 2-Hydroxy-2-hexyloctansäure, 2-Hydroxy-2-heptyloctansäure, 3-Hydroxypropansäure, 3-Hydroxybutansäure, 3-Hydroxypentansäure, 3-Hydroxyhexansäure, 3-Hydroxyheptansäure, 3-Hydroxyoctansäure, 3-Hydroxy-3-methylbutansäure, 3-Hydroxy-3-methylpentansäure, 3-Hydroxy-3-ethylpentansäure, 3-Hydroxy-3-methylhexansäure, 3-Hydroxy-3-ethylhexansäure, 3-Hydroxy-3-propylhexansäure, 3-Hydroxy-3-methylheptansäure, 3-Hydroxy-3-ethylheptansäure, 3-Hydroxy-3-propylheptansäure, 3-Hydroxy-3-butylheptansäure, 3-Hydroxy-3-methyloctansäure, 3-Hydroxy-3-ethyloctansäure, 3-Hydroxy-3-propyloctansäure, 3-Hydroxy-3-pentyloctansäure, 4-Hydroxybutansäure, 4-Hydroxypentansäure, 4-Hydroxyhexansäure, 4-Hydroxyheptansäure, 4-Hydroxyoctansäure, 4-Hydroxy-4-methylpentansäure, 4-Hydroxy-4-Methylhexansäure, 4-Hydroxy-4-ethylhexansäure, 4-Hydroxy-4-metylheptansäure, 4-Hydroxy-4-ethylheptansäure, 4-Hydroxy-4-propylheptansäure, 4-Hydroxy-4-methyloctansäure, 4-hydroxy-4-Ethyloctansäure, 4-Hydroxy-4-propyloctansäure, 4-Hydroxy-4-butyloctansäure, 5-Hydroxypentansäure, 5-Hydroxyhexansäure, 5-Hydroxyheptansäure, 5-Hydroxyoctansäure, 5-Hydroxy-5-methylhexansäure, 5-Hydroxy-5-methylheptansäure, 5-Hydroxy-5-ethylheptansäure, 5-Hydroxy-5-methyloctansäure, 5-Hydroxy-5-ethyloctansäure, 5-Hydroxy-5-propyl-octansäure, 6-Hydroxyhexansäure, 6-Hydroxyheptansäure, 6-Hydroxyoctansäure, 6-Hydroxy-6-methylheptansäure, 6-Hydroxy-6-methyloctansäure, 6-Hydroxy-6-ethyloctansäure, 7-Hydroxyheptansäure, 7-Hydroxyoctansäure, 7-Hydroxy-7-methyloctansäure und 8-Hydroxyoctansäure.

13. Verfahren nach einem der vorhergehenden Ansprüche, **dadurch gekennzeichnet, dass** nach Schritt b) eine Aufreinigung des erhaltenen intramolekularen Diesters der in Schritt a) eingesetzten Hydroxycarbonsäure erfolgt, z.B. durch Destillation und/oder Kristallisation.

14. Verfahren zur Herstellung einer Polyhydroxycarbonsäure, **dadurch gekennzeichnet, dass** im Anschluss an ein Verfahren nach einem der vorhergehenden Ansprüche der intramolekulare Diester der in Schritt a) eingesetzten Hydroxycarbonsäure in einer ringöffnenden Polymerisation eingesetzt wird.

## Claims

1. A method for producing an intramolecular diester of a hydroxycarboxylic acid, comprising the following steps:
a) polycondensing a hydroxycarboxylic acid to an oligomeric hydroxycarboxylic acid, and
b) cyclizing depolymerization of the oligomeric hydroxycarboxylic acid to obtain an intramolecular diester of the hydroxycarboxylic acid used in step a),
**characterized in that**
in step b), a plastic recyclate is added, comprising or consisting of at least one polyhydroxy carboxylic acid, wherein at least one polyhydroxy carboxylic acid is derived from the hydroxycarboxylic acid used in step a); and/or
at least one co-polyhydroxy carboxylic acid, wherein at least one co-polyhydroxy carboxylic acid is derived from the hydroxycarboxylic acid used in step a) as well as at least one hydroxycarboxylic acid different from the hydroxycarboxylic acid used in step a).

2. The method according to claim 1, **characterized in that** the plastic recyclate is added in the form of a melt, for example by means of an extruder.

3. The method according to any one of the preceding claims, **characterized in that** the plastic recyclate is added in step b), based on the oligomeric hydroxycarboxylic acid, up to a maximum of 50 wt.%, preferably 0.01 to 40 wt.%, further preferably 0.1 to 30 wt.%, further preferably 1 to 25 wt.%, further preferably 2.5 to 20 wt.%, and particularly preferably 5 to 15 wt.%.

4. The method according to any one of the preceding claims, **characterized in that** the step of cyclizing depolymerization b) is carried out at temperatures between 200 and 250°C, preferably between 210 and 230°C.

5. The method according to any one of the preceding claims, **characterized in that** plastic recyclate comprises or consists of
a polyhydroxy carboxylic acid or a co-polyhydroxy carboxylic acid;
a blend, comprising or consisting of at least one polyhydroxy carboxylic acid and/or at least one co-polyhydroxy carboxylic acid as well as at least one plastic material different from the at least one polyhydroxy carboxylic acid and the at least one co-polyhydroxy carboxylic acid, or
a blend, comprising or consisting of at least two different polyhydroxy carboxylic acid or at least two different co-polyhydroxy carboxylic acids.

6. The method according to one of the two preceding claims, **characterized in that** the at least one plastic material different from the at least one polyhydroxy carboxylic acid and the at least one co-polyhydroxy carboxylic acid is selected from the group consisting of polyesters, in particular polybutylene succinate (PBS), polybutylene adipate-co-terephthalate (PBAT, Ecoflex^{®}), polybutylene succinate-co-adipate, poly-hydroxyalkanoate (PHA), polyethylene terephthalate (PET), polybutylene terephthalate (PBT), polytrimethylene terephthalate (PTT); polyamides; polyvinyl acetate; polyolefins, in particular polyethylene (PE) and/or polypropylene (PP); ethylene-vinyl acetate copolymers; polycarbonates; polyvinyl alcohol (PVA); polyethers, in particular polyethylene glycol (PEG), polytrimethylene glycol (PTMG), polypropylene glycol (PPG) or copolymers thereof; polystyrene; acrylonitrile-butadienestyrene copolymers; polyacetates; poly(meth)acrylates; as well as thermoplastic and thermoset elastomers.

7. The method according to any one of the preceding claims, **characterized in that** the at least one further plastic material accrue as a bottom component.

8. The method according to any one of the preceding claims, **characterized in that** the intramolecular diester of the hydroxycarboxylic acid obtained in step b) is separated in the gaseous state of matter.

9. The method according to any one of the preceding claims, **characterized in that**, in the event that the plastic recyclate added in step b) additionally comprises at least one polyhydroxy carboxylic acid that is not derived from the hydroxycarboxylic acid used in step a),
at least one co-polyhydroxy carboxylic acid, wherein at least one co-polyhydroxy carboxylic acid is derived from the hydroxycarboxylic acid used in step a) as well as at least one hydroxycarboxylic acid different from the hydroxycarboxylic acid used in step a); and/or
additionally at least one co-polyhydroxy carboxylic acid, wherein at least one co-polyhydroxy carboxylic acid is exclusively derived from at least two hydroxycarboxylic acids different from the hydroxycarboxylic acid used in step a),
a mixture is obtained, comprising the intramolecular diester of the hydroxycarboxylic acid used in step a), an intramolecular diester of the hydroxycarboxylic acid used in step a) and of a hydroxycarboxylic acid different from the hydroxycarboxylic acid used in step a), as well as an intramolecular diester of at least one hydroxycarboxylic acid different from the hydroxycarboxylic acid used in step a), and
the intramolecular diester of the hydroxycarboxylic acid used in step a) is separated from the mixture, preferably by means of distillation.

10. The method according to any one of the preceding claims, **characterized in that** an alcohol having a boiling point, determined under standard conditions (IUPAC, 1982), of ≥ 150°C is additionally added in step b), preferably in an amount of 0.01 to 2 wt.%, particularly preferably of 0.1 to 0.5 wt.%, based on the total amount of compounds used in step b).

11. The method according to the preceding claim, **characterized in that** the alcohol is selected from the group consisting of alkane diols, in particular ethylene glycol, propylene glycol, and/or butylene glycol; fatty alcohols having 6 to 22 carbon atoms, in particular decanol; alkane triols, cyclic alcohols, as well as mixtures and combinations thereof.

12. The method according to any one of the preceding claims, **characterized in that** the hydroxycarboxylic acid used in step a) is selected from the group consisting of 2-hydroxypropanoic acid, 2-hydroxyethanoic acid, 2-hydroxybutanoic acid, 2-hydroxypentanoic acid, 2-hydroxyhexanoic acid, 2-hydroxyheptanoic acid, 2-hydroxyoctanoic acid, 2-hydroxy-2-methylpropanoic acid, 2-hydroxy-2-methylbutanoic acid, 2-hydroxy-2-ethylbutanoic acid, 2-hydroxy-2-methylpentanoic acid, 2-hydroxy-2-ethylpentanoic acid, 2-hydroxy-2-propylpentanoic acid, 2-hydroxy-2-butylpentanoic acid, 2-hydroxy-2-methylhexanoic acid, 2-hydroxy-2-ethylhexanoic acid, 2-hydroxy-2-propylhexanoic acid, 2-hydroxy-2-butylhexanoic acid, 2-hydroxy-2-pentylhexanoic acid, 2-hydroxy-2-methylheptanoic acid, 2-hydroxy-2-ethylheptanoic acid, 2-hydroxy-2-propylheptanoic acid, 2-hydroxy-2-butylheptanoic acid, 2-hydroxy-2-pentylheptanoic acid, 2-hydroxy-2-hexylheptanoic acid, 2-hydroxy -2-methyloctanoic acid, 2-hydroxy-2-ethyloctanoic acid, 2-hydroxy-2-propyloctanoic acid, 2-hydroxy-2-butyloctanoic acid, 2-hydroxy-2-pentyloctanoic acid, 2-hydroxy-2-hexyloctanoic acid, 2-hydroxy-2-heptyloctanoic acid, 3-hydroxypropanoic acid, 3-hydroxybutanoic acid, 3-hydroxypentanoic acid, 3-hydroxyhexanoic acid, 3-hydroxyheptanoic acid, 3-hydroxyoctanoic acid, 3-hydroxy-3-methylbutanoic acid, 3-hydroxy-3-methylpentanoic acid, 3-hydroxy-3-ethylpentanoic acid, 3-hydroxy-3-methylhexanoic acid, 3-hydroxy-3-ethylhexanoic acid, 3-hydroxy-3-propylhexanoic acid, 3-hydroxy-3-methylheptanoic acid, 3-hydroxy-3-ethylheptanoic acid, 3-hydroxy-3-propylheptanoic acid, 3-hydroxy-3-butylheptanoic acid, 3-hydroxy-3-methyloctanoic acid, 3-hydroxy-3-ethyloctanoic acid, 3-hydroxy-3-propyloctanoic acid, 3-hydroxy-3-pentyloctanoic acid, 4-hydroxybutanoic acid, 4-hydroxypentanoic acid, 4-hydroxyhexanoic acid, 4-hydroxyheptanoic acid, 4-hydroxyoctanoic acid, 4-hydroxy-4-methylpentanoic acid, 4-hydroxy-4-methylhexanoic acid, 4-hydroxy-4-ethylhexanoic acid, 4-hydroxy-4-methylheptanoic acid, 4-hydroxy-4-ethylheptanoic acid, 4-hydroxy-4-propylheptanoic acid, 4-hydroxy-4-methyloctanoic acid, 4-hydroxy-4-ethyloctanoic acid, 4-hydroxy-4-propyloctanoic acid, 4-hydroxy-4-bu-tyloctanoic acid, 5-hydroxypentanoic acid, 5-hydroxyhexanoic acid, 5-hydroxyheptanoic acid, 5-hydroxyoctanoic acid, 5-hydroxy-5-methylhexanoic acid, 5-hydroxy-5-methylheptanoic acid, 5-hydroxy-5-ethylheptanoic acid, 5-hydroxy-5-methyloctanoic acid, 5-hydroxy-5-ethyloctanoic acid, 5-hydroxy-5-propyloctanoic acid, 6-hydroxyhexanoic acid, 6-hydroxyheptanoic acid, 6-hydroxyoctanoic acid, 6-hydroxy-6-methylheptanoic acid, 6-hydroxy-6-methyloctanoic acid, 6-hydroxy-6-ethyloctanoic acid, 7-hydroxyheptanoic acid, 7-hydroxyoctanoic acid, 7-hydroxy-7-methyloctanoic acid and 8-hydroxyoctanoic acid.

13. The method according to any one of the preceding claims, **characterized in that**, after step b), a purification of the obtained intramolecular diester of the hydroxycarboxylic acid used in step a) is carried out, for example by distillation and/or crystallization.

14. A method for producing a polyhydroxycarboxylic acid, **characterized in that**, subsequent to a method according to any one of the preceding claims, the intramolecular diester of the hydroxycarboxylic acid used in step a) is used in a ring-opening polymerization process.

## Revendications

1. Procédé de préparation d'un diester intramoléculaire d'un acide hydroxycarboxylique, comprenant les étapes suivantes :
a) la polycondensation d'un acide hydroxycarboxylique en un acide hydroxycarboxylique oligomère, et
b) la dépolymérisation cyclisante de l'acide hydroxycarboxylique oligomère pour obtenir un diester intramoléculaire de l'acide hydroxycarboxylique utilisé dans l'étape a),
**caractérisé en ce que**
dans l'étape b), un recyclat de matière plastique, contenant ou constitué
d'au moins un acide polyhydroxycarboxylique, dans lequel au moins un acide polyhydroxycarboxylique est dérivé de l'acide hydroxycarboxylique utilisé dans l'étape a), et/ou
d'au moins un acide co-polyhydroxycarboxylique, dans lequel au moins un acide co-polyhydroxycarboxylique est dérivé de l'acide hydroxycarboxylique utilisé dans l'étape a) ainsi que d'au moins un acide hydroxycarboxylique différent de l'acide hydroxycarboxylique utilisé dans l'étape a),
est ajouté.

2. Procédé selon la revendication 1, **caractérisé en ce que** le recyclat de plastique est ajouté sous forme de masse fondue, par exemple au moyen d'une extrudeuse.

3. Procédé selon l'une quelconque des revendications précédentes, **caractérisé en ce que** le recyclat de plastique est ajouté dans l'étape b), par rapport à l'acide hydroxycarboxylique oligomère, jusqu'à un maximum de 50 % en poids, de préférence de 0,01 à 40 % en poids, de préférence encore de 0,1 à 30 % en poids, de préférence encore de 1 à 25 % en poids, de préférence encore de 2,5 à 20 % en poids, de manière particulièrement préférée de 5 à 15 % en poids.

4. Procédé selon l'une quelconque des revendications précédentes, **caractérisé en ce que** l'étape de dépolymérisation cyclisante b) est réalisée à des températures comprises entre 200 et 250 °C, de préférence entre 210 et 230 °C.

5. Procédé selon l'une quelconque des revendications précédentes, **caractérisé en ce que** le recyclat de matière plastique comporte
un acide polyhydroxycarboxylique ou un acide co-polyhydroxycarboxylique,
un mélange contenant ou constitué d'au moins un acide polyhydroxycarboxylique et/ou d'au moins un acide co-polyhydroxycarboxylique ainsi qu'au moins une matière plastique différente de l'au moins un acide polyhydroxycarboxylique et de l'au moins un acide co-polyhydroxycarboxylique, ou
un mélange contenant ou constitué d'au moins deux acides polyhydroxycarboxyliques différents ou d'au moins deux acides co-polyhydroxycarboxyliques différents,
ou en est constitué.

6. Procédé selon l'une des deux revendications précédentes, **caractérisé en ce que** la au moins une matière plastique différente du au moins un acide polyhydroxycarboxylique et du au moins un acide co-polyhydroxycarboxylique est choisie dans le groupe constitué par les polyesters, en particulier le polybutylène succinate (PBS), le polybutylène adipate-co-téréphtalate (PBAT, Ecoflex^{®}), le polybutylène succinate-co-adipate, les polyhydroxyalcanoates (PHA), le polyéthylène téréphtalate (PET), le polybutylène téréphtalate (PBT), le polytriméthylène téréphtalate (PTT) ; les polyamides ; l'acétate de polyvinyle ; les polyoléfines, en particulier le polyéthylène (PE) et/ou polypropylène (PP) ; les copolymères éthylène-acétate de vinyle ; les polycarbonates ; l'alcool polyvinylique (PVA) ; les polyéthers, en particulier le polyéthylèneglycol (PEG), le polytriméthylèneglycol (PTMG), le polypropylèneglycol (PPG) ou les copolymères de ceux-ci ; le polystyrène ; les copolymères acrylonitrile-butadiène-styrène ; les polyacétates ; les poly(méth)acrylates ; ainsi que des élastomères thermoplastiques et thermodurcissables.

7. Procédé selon l'une quelconque des revendications précédentes, **caractérisé en ce que** la au moins une autre matière plastique est produite en tant que composant de fond de cuve.

8. Procédé selon l'une quelconque des revendications précédentes, **caractérisé en ce que** le diester intramoléculaire de l'acide hydroxycarboxylique obtenu à l'étape b) est séparé à l'état d'agrégat gazeux.

9. Procédé selon l'une quelconque des revendications précédentes, **caractérisé en ce que**, dans le cas où le recyclat de matière plastique ajouté dans l'étape b) contient
en outre, au moins un acide polyhydroxycarboxylique qui n'est pas dérivé de l'acide hydroxycarboxylique utilisé dans l'étape a),
un acide co-polyhydroxycarboxylique, au moins un acide co-polyhydroxycarboxylique est dérivé de l'acide hydroxycarboxylique utilisé dans l'étape a) ainsi que d'au moins un acide hydroxycarboxylique différent de l'acide hydroxycarboxylique utilisé dans l'étape a), et/ou
en outre, au moins un acide co-polyhydroxycarboxylique, dans lequel au moins un acide co-polyhydroxycarboxylique est exclusivement dérivé d'au moins deux acides hydroxycarboxyliques différents de l'acide hydroxycarboxylique utilisé dans l'étape a),
un mélange contenant le diester intramoléculaire de l'acide hydroxycarboxylique utilisé dans l'étape a), un diester intramoléculaire de l'acide hydroxycarboxylique utilisé dans l'étape a) et d'un acide hydroxycarboxylique différent de l'acide hydroxycarboxylique utilisé dans l'étape a), ainsi qu'un diester intramoléculaire composé d'au moins un acide hydroxycarboxylique différent de l'acide hydroxycarboxylique utilisé dans l'étape a), est obtenu, et
le diester intramoléculaire de l'acide hydroxycarboxylique utilisé dans l'étape a) est séparé du mélange, de préférence par distillation.

10. Procédé selon l'une quelconque des revendications précédentes, **caractérisé en ce que**, dans l'étape b), un alcool avec un point d'ébullition déterminé dans des conditions standard (IUPAC, 1982) ≥ 150 °C est en outre ajouté, de préférence en une quantité de 0,01 à 2 % en poids, de manière particulièrement préférée de 0,1 à 0,5 % en poids, par rapport à la quantité totale des composés utilisés dans l'étape b).

11. Procédé selon la revendication précédente, **caractérisé en ce que** l'alcool est choisi dans le groupe constitué par les alcanediols, en particulier l'éthylèneglycol, le propylèneglycol, et/ou le butylèneglycol ; les alcools gras avec 6 à 22 atomes de carbone, en particulier le décanol ; les alcanetriols, les alcools cycliques, ainsi que les mélanges et les combinaisons de ceux-ci.

12. Procédé selon l'une quelconque des revendications précédentes, **caractérisé en ce que** l'acide hydroxycarboxylique utilisé dans l'étape a) est choisi dans le groupe constitué par l'acide 2-hydroxypropanoïque, l'acide 2-hydroxyéthanoïque, l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-2-méthylpropanoïque, l'acide 2-hydroxy-2-méthylbutanoïque, l'acide 2-hydroxy-2-éthylbutanoïque, l'acide 2-hydroxy-2-méthylpentanoïque, l'acide 2-hydroxy-2-éthylpentanoïque, l'acide 2-hydroxy-2-propylpentanoïque, l'acide 2-hydroxy-2-butylpentanoïque, l'acide 2-hydroxy-2-méthylhexanoïque, l'acide 2-hydroxy-2-éthylhexanoïque, l'acide 2-hydroxy-2-propylhexanoïque, l'acide 2-hydroxy-2-butylhexanoïque, l'acide 2-hydroxy-2-pentylhexanoïque, l'acide 2-hydroxy-2-méthylheptanoïque, l'acide 2-hydroxy-2-éthylheptanoïque, l'acide 2-hydroxy-2-propylheptanoïque, l'acide 2-hydroxy-2-butylheptanoïque, l'acide 2-hydroxy-2-pentylheptanoïque, l'acide 2-hydroxy-2-hexylheptanoïque, l'acide 2-hydroxy-2-méthyloctanoïque, l'acide 2-hydroxy-2-éthyloctanoïque, l'acide 2-hydroxy-2-propyloctanoïque, l'acide 2-hydroxy-2-butyloctanoïque, l'acide 2-hydroxy-2-pentyloctanoïque, l'acide 2-hydroxy-2-hexyloctanoïque, l'acide 2-hydroxy-2-heptyloctanoïque, l'acide 3-hydroxypropanoïque, l'acide 3-hydroxybutanoïque, l'acide 3-hydroxypentanoïque, l'acide 3-hydroxyhexanoïque, l'acide 3-hydroxyheptanoïque, l'acide 3-hydroxyoctanoïque, l'acide 3-hydroxy-3-méthylbutanoïque, l'acide 3-hydroxy-3-méthylpentanoïque, l'acide 3-hydroxy-3-éthylpentanoïque, l'acide 3-hydroxy-3-méthylhexanoïque, l'acide 3-hydroxy-3-éthylhexanoïque, l'acide 3-hydroxy-3-propylhexanoïque, l'acide 3-hydroxy-3-méthylheptanoïque, l'acide 3-hydroxy-3-éthylheptanoïque, l'acide 3-hydroxy-3-propylheptanoïque, l'acide 3-hydroxy-3-butylheptanoïque, l'acide 3-hydroxy-3-méthyloctanoïque, l'acide 3-hydroxy-3-éthyloctanoïque, l'acide 3-hydroxy-3-propyloctanoïque, l'acide 3-hydroxy- 3-pentyloctanoïque, l'acide 4-hydroxybutanoïque, l'acide 4-hydroxypentanoïque, l'acide 4-hydroxyhexanoïque, l'acide 4-hydroxyheptanoïque, l'acide 4-hydroxyoctanoïque, l'acide 4-hydroxy-4-méthylpentanoïque, l'acide 4-hydroxy-4-méthylhexanoïque, l'acide 4-hydroxy-4-éthylhexanoïque, l'acide 4-hydroxy-4-métylheptanoïque, l'acide 4-hydroxy-4-éthylheptanoïque, l'acide 4-hydroxy-4-propylheptanoïque, l'acide 4-hydroxy-4-méthyloctanoïque, l'acide 4-hydroxy-4-éthyloctanoïque, l'acide 4-hydroxy-4propyloctanoïque, l'acide 4-hydroxy-4-butyloctanoïque, l'acide 5-hydroxypentanoïque, l'acide 5-hydroxyhexanoïque, l'acide 5-hydroxyheptanoïque, l'acide 5-hydroxyoctanoïque, l'acide 5-hydroxy-5-méthylhexanoïque, l'acide 5-hydroxy-5-méthylheptanoïque, l'acide 5-hydroxy-5-éthylheptanoïque, l'acide 5-hydroxy-5-méthyloctanoïque, l'acide 5-hydroxy-5-éthyloctanoïque, l'acide 5-hydroxy-5-propyloctanoïque, l'acide 6-hydroxyhexanoïque, l'acide 6-hydroxyheptanoïque, l'acide 6-hydroxyoctanoïque, l'acide 6-hydroxy-6-méthylheptanoïque, l'acide 6-hydroxy-6-méthyloctanoïque, l'acide 6-hydroxy-6-éthyloctanoïque, l'acide 7-hydroxyheptanoïque, l'acide 7-hydroxyoctanoïque, l'acide 7-hydroxy-7-méthyloctanoïque et l'acide 8-hydroxyoctanoïque.

13. Procédé selon l'une quelconque des revendications précédentes, **caractérisé en ce que**, après l'étape b), une purification du diester intramoléculaire obtenu de l'acide hydroxycarboxylique utilisé dans l'étape a) s'effectue, par exemple par distillation et/ou cristallisation.

14. Procédé de préparation d'un acide polyhydroxycarboxylique, **caractérisé en ce que**, à la suite d'un procédé selon l'une quelconque des revendications précédentes, le diester intramoléculaire de l'acide hydroxycarboxylique utilisé dans l'étape a) est utilisé dans une polymérisation par ouverture de cycle.
